(19) Europäisches Patentamt
European Patent Office
Office européen des brevets

(11) Numéro de publication: **0 061 386**
**B1**

(12) # FASCICULE DE BREVET EUROPEEN

(45) Date de publication du fascicule du brevet: **20.06.84**

(51) Int. Cl.³: **C 07 D 333/36,** A 61 K 31/38

(21) Numéro de dépôt: **82400433.7**

(22) Date de dépôt: **11.03.82**

(54) **Acides ((2-oxo-3-tétrahydrothiénylcarbamoyl)-alkylthio) acétiques, leurs sels et leurs esters, leur procédé de préparation et les compositions pharmaceutiques en contenant.**

(30) Priorité: **19.03.81 FR 8105496**

(43) Date de publication de la demande:
**29.09.82 Bulletin 82/39**

(45) Mention de la délivrance du brevet:
**20.06.84 Bulletin 84/25**

(84) Etats contractants désignés:
**AT BE CH DE FR GB IT LI LU NL SE**

(56) Documents cités:
**DE - B - 1 177 001**
**FR - A - 1 451 177**
**FR - A - 2 129 877**
**FR - A - 2 147 468**
**FR - A - 2 265 355**
**FR - A - 2 313 032**

(73) Titulaire: **REFARMED, Recherches Pharmaceutiques et Medicales S.A.**
**Dammstrasse 29**
**CH-8702 Zollikon (CH)**

(72) Inventeur: **Gonella, Jacques**
**29 Bahnhofstrasse**
**CH-8702 Zollikon (ZH) (CH)**

(74) Mandataire: **Combe, André et al,**
**CABINET BEAU DE LOMENIE 55 rue d'Amsterdam**
**F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 061 386 B1

**Description**

La présente invention concerne des acides [(2-oxo-3-tétrahydrothiénylcarbamoyl)-alkylthio]acétiques ayant une activité mucolytique, un procédé pour leur préparation et des compositions pharmaceutiques les renfermant en tant qu'ingrédients actifs.

Plus particulièrement, selon un de ses aspects, l'invention se réfère à des composés de formule générale suivante:

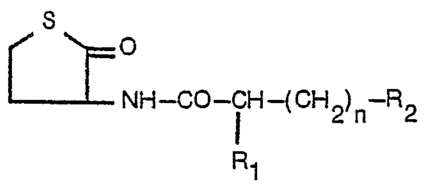

I

dans laquelle n représente 0 ou 1 et un des substituants $R_1$ et $R_2$ représente un atome d'hydrogène ou un groupe méthyle et l'autre représente un groupe $S—CH_2—COOR_3$, où $R_3$ est un atome d'hydrogène ou d'un métal alcalin ou bien un groupe $R_3'$, $R_3'$ étant alkyle inférieur ou bien un groupe

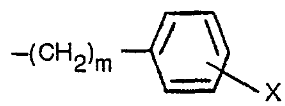

où m représente 0 ou 1 et X représente un atome d'hydrogène, ou un groupe alkyle inférieur, alcoxy inférieur ou nitro

Le terme "alkyle inférieur", tel qu'indiqué ici, désigne un groupe alkyle ayant de 1 à 3 atomes de carbone, à savoir les groupes méthyle, éthyle, propyle et isopropyle. Les termes "alcoxy inférieur" et "alkylthio inférieur" désignent les groupes hydroxyle et sulfhydryle où l'atome d'hydrogène est remplace par un groupe alkyle inférieur tel que désigné ci-dessus.

Selon un autre de ses aspects, la présente invention se réfère à un procédé pour la préparation d'acides [(2-oxo-3-terahydrothiénylcarbamoyl)-alkylthio]acétiques de formule I, caractérisé en ce qu'on fait réagir un 3-haloacylamino-2-oxo-tétrahydrothiophène de formule:

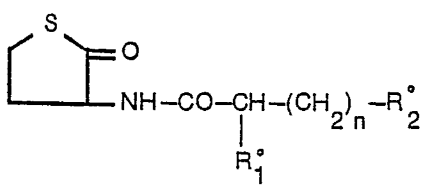

II

où un des $R_1^o$ et $R_2^o$ est un atome d'hydrogène ou un groupe méthyle et l'autre un atome de chlore ou de brome, avec un acide thioglycolique de formule:

$$HS—CH_2—COOR_3^o \qquad III$$

où $R_3^o$ a la signification donnée ci-dessus pour $R_3$, mais est autre qu'un métal alcalin, en présence d'un accepteur de protons à une température de 15 à 120°C et, lorsqe $R_3^o$ est de l'hydrogène, on transforme éventuellement le produit ainsi obtenu dans ses sels alcalins.

Comme accepteur de protons, ou peut utiliser une base inorganique telle que l'hydroxyde de sodium ou de potassium, le carbonate de sodium ou de potassium, le bicarbonate de sodium ou de potassium, ainsi qu'une base organique telle que la triméthylamine, la triéthylamine ou la pipéridine.

Comme diluant de réaction, ou peut utiliser de l'eau ou bien un solvant organique. De préference, on opère dans de l'eau en présence d'hydroxyde de sodium ou de potassium.

La réaction est rapide et sa durée de (2 à 30 min) dépend de la température. En général, après 5 min de chauffage au reflux en solution aqueuse, la réaction est complète et le produit final est isolé selon les techniques habituelles, par exemple par acidification et filtration ou par acidification et extraction avec un solvant approprié.

Si, comme produit intermédiaire de formule III, on utilise l'acide thioglycolique libre ($R_3^o = H$), on obtient un acide libre de formule I ($R_3 = H$) qui peut être salifié selon les techniques habituelles pour obtenir un sel alcalin ($R_3 =$ méthyl alcalin).

Selon une variante du procédé de la présente invention, l'acide libre de formule I ($R_3 = H$) peut être estérifié avec un composé de formule $R_3'—OH$, où $R_3'$ est tel que défini ci-dessus, selon les techniques conventionnelles.

L'estérification est conduite en faisant réagir un dérivé fonctionnel dudit acide libre, comme le chlorure ou un anhydride mixte, avec le composé $R_3'$—OH à une température de –20 à +40°C en présence d'un accepteur de protons dans un solvant organique inerte.

Selon un mode opératoire préférentiel, on traite l'acide libre avec du chlorocarbonate d'éthyle et avec le composé $R_3'$—OH en présence d'une base organique, la triéthylamine de préférence, à une température de –15 à –10°C.

Comme solvant organique, on utilise de préférence un hydrocarbure, notamment hexane, benzène ou toluène, mais d'autres solvants inertes dans les conditions d'estérification, comme le dioxane, sont également convenables.

L'ester résultant est isolé d'une façon classique, par élimination des impuretés, évaporation du solvant et cristallisation.

Les acides [(2-oxo-3-tétrahydrothiénylcarbamoyl)-alkylthio]acétiques de la présente invention possèdent des propriétés pharmacologiques intéressantes sans présenter de toxicité.

En particulier, les composés de la présente invention sont capables de provoquer une augmentation de la production de mucus bronchique chez les animaux, comme il a été démontré dans un essai d'activité chez le lapin.

On a utilisé des lapins pesant 3 kg environ avec une canule implantée dans la trachée selon la technique de SCURI et al., Boll. Chim. Farm., *119*, (3), 1980. La quantité de mucus produit et extrété a été enregistrée les 4 h précédant et les 4 h suivant le traitement.

Le tableau I ci-dessous montre les résultats obtenus avec un composé représentatif de la présente invention, l'acide [(2-oxo-3-tétrahydrothiénylcarbamoyl)-méthylthio]acétique, ci-après indiqué par son numéro de code PV 144. Un produit à action mucolytique bien connu, la S-carboxyméthylcystéine, ci-après indiquée par sa Dénomination Commune Internationale "carbocystéine", a été utilisé comme produit de référence. Les produits ont été administrés par voie intraveineuse et les résultats sont exprimés en pourcentage de variation de sécrétion de mucus par rapport aux contrôles ayant reçu de la solution physiologique.

TABLEAU I

| Produit | dose | variation % |
|---|---|---|
| PV 144 | 10 mg/kg<br>(0,04 mmol/kg) | 52,1 |
| Carbocystéine | 10 mg/kg<br>(0,055 mmol/kg) | 51,8 |

De ce tableau, il ressort que le produit de la présente invention est au moins aussi actif que le produit de référence à la même dose pondérale qui correspond à une dose molaire de PV 144 inférieure de 37,5% à celle du composé de référence.

Dans le test d'activité mucolytique basé sur l'inhalation $SO_2$ chez le rat, les composés de la présente invention ont montré une bonne activité mucolytique. En particulier, le PV 144 à la dose orale de 500 mg/kg (2 mmol/kg) a comporté une régression significative des dommages provoqués sur les bronches par l'action du dioxyde de soufre. Cette régression est qualitativement et quantitativement égale à celle obtenue par un dose de 500 mg/kg (3,35 mmol/kg) de carbocystéine.

Le tableau II ci-dessous montre les données de toxicité aiguë déterminées chez le rat et la souris par les voies orale et intraveineuse sur un composé représentatif de la présente invention, le PV 144.

TABLEAU II

| | PV 144 $DL_{50}$ (mg/kg) | |
|---|---|---|
| Animal | os | i.v. |
| Souris | >10 000 | >3 500 |
| Rat | >10 000 | >3 500 |

Le tableau montre que le produit représentatif de la présente invention est pratiquement dépourvu de toxicité.

Grâce à leur activité mucolytique, les acides [(2-oxo-3-tétrahydrothiénylcarbamoyl)-alkylthio]acétiques de la présente invention peuvent être utiles pour le traitement des maladies de l'appareil respiratoire; en particulier, ils peuvent être employés en pneumologie dans les bronchites, dans les trachéobronchites, dans les pharyngites, dans les rhinopharyngites, dans les otites et les sinusites.

Les produits de la présente invention peuvent être administrés par la voie orale ou parentérale, rectale ou par inhalation soit seuls, soit sous forme de préparations pharmaceutiques appropriées, telles que des comprimés, des poudres, des granules, des capsules, des élixirs, des suspensions, des sirops, des suppositoires et des solutions ou des suspensions pour injection ou pour inhalation.

Les préparations pharmaceutiques destinées à l'administration par voie orale contiennent, ontre la substance active, un ou plusieurs excipients organiques ou minéraux acceptables du point de vue pharmaceutique et compatibles avec le principe actif, ainsi que des édulcorants, des aromatisants, des colorants, des agents de conservation et similaires.

Pour la préparation des comprimés, on pourra utiliser, comme excipients, des diluants inertes comme le carbonate de calcium, le carbonate de sodium, le lactose, le talc, des agents de granulation et de désagrégation comme l'amidon et l'acide alginique, des liants comme l'amidon, la gélatine et la gomme arabique, des agents lubrifiants comme le stéarate de magnésium et l'acide stéarique. Les comprimés peuvent être revêtus on non.

Le revêtement a pour de retarder la décomposition et l'absorption de la substance active dans le tractus gastro-intestinal et de produire ainsi un effet retard prolongé. Les suspensions, les sirops et les élixirs peuvent contenir, ontre la substance active, des agents de suspension, tels que la méthylcellulose, la gomme adragante, l'alginate de sodium et similaires, des mouillants, tels que la lécithine, le stéarate de polyoxyéthylène, le mono-oléate de polyoxyéthylènesorbitanne et des agents de conservation, tels que le p-hydroxybenzoate d'éthyle. Les gélules peuvent contenir la substance active soit seule, soit en mélange avec des diluants inertes solides comme, par exemple, la carbonate de calcium, le phosphate de calcium et le kaolin.

On prépare des fluides pour administration orale, sous des formes posologiques unitaires, tels que sirops où chaque cuillère à café de composition contient une quantité prédéterminée du composé actif à administrer.

On prépare un sirop en mettant l'ingrédient actif en suspension dans une solution aqueuse de saccharose convenablement parfumée, par exemple par de la vanilline ou de l'essence de citron.

On prépare des granules destinés à la reconstitution d'une préparation liquide orale en utilisant des diluants solubles dans l'eau. On mouille le composé actif et un diluant soluble dans l'eau tel que le saccharose, glucose, etc., avec un liant tel que mucilage d'acacia, solution de gélatine, solution de méthylcellulose et on fait passer sur un tamis en forçant pour former des granules que l'on fait sécher. Il est avantageux d'introduire dans la composition un agent de mise en suspension comme la gomme adragante.

Les granules ainsi obtenus peuvent être conditionnés dans des sachets et ainsi administrés sous forme posologique unitaire.

Pour l'administration parentérale, les compositions selon l'invention peuvent se présenter sous forme d'ampoules contenant le principe actif dissous dans un solvant pour injection, eau pour injection ou une solution physiologique de préférence. L'unité de dosage peut aussi être présentée sous forme d'ampoule stérile pour la préparation de solutions extemporanées pour l'administration parentérale.

Pour l'administration par inhalation, les compositions selon l'invention peuvent se présenter sous forme d'une poudre ou d'un spray aérosol; on peut aussi utiliser les mêmes ampoules qu'on utilise pour l'administration parentérale.

Les compositions de la présente invention peuvent également renfermer d'autres produits actifs tels que, par exemple,des antibiotiques, des bronchodilatateurs, des antitussifs,des balsamiques, des anti-histaminiques ou des anesthésiques locaux.

Les compositions de la présente invention contiennent 1 à 1000 mg d'ingrédient actif par unité de dosage et peuvent être administrées de façon à fournir une dose de substance active variable de 4 à 2000 mg par jour.

Les exemples suivants illustrent l'invention sans toutefois la limiter.

## Exemple 1

A une solution de 26,5 g d'hydroxyde de sodium dans 470 ml d'eau, on ajoute, sous atmosphère d'azote, 30,5 g d'acide thioglycolique et puis 64 g de 3-chloroacétamido-2-oxo-tétrahydrothiophène. On chauffe au reflux pendant 5 min, puis on refroidit, on acidifie avec de l'acide chlorhydrique concentré jusqu'a pH 1 à 2 et l'on filtre.

Par cristallisation avec 500 ml d'éthanol, on obtient 40 g d'acide [(2-oxo-3-tétrahydrothiényl-carbamoyl)-méthylthio]acétique (PV 144), F. 156 à 158°C.

A une solution de 24,9 g du produit ainsi obtenu dans 50 ml d'eau, on ajoute 5,3 g de carbonate de sodium, puis on évapore à sec la solution limpide ainsi obtenue et on cristallise le résidu avec 150 ml d'éthanol à 85%. On obtient 28 g de [(2-oxo-3-tétrahydrothiénylcarbamoyl)-méthylthio]acétate de sodium.

De même, à partir de 24,9 g d'acide [(2-oxo-3-tétrahydrothiénylcarbamoyl)-méthylthio]acétique et de 6,9 g de carbonate de potassium, on obtient 28 g de [(2-oxo-3-tétrahydrothiénylcarbamoyl)-méthylthio]acétate de potassium.

Exemple 2

A une solution de 32 g d'hydroxyde de sodium dans 800 ml d'eau, on ajoute, sous atmosphère d'azote, 37 g d'acide thioglycolique et, ensuite, 101 g de 3-(2-bromopropionamido)-2-oxo-tétrahydrothiophène, puis on chauffe 5 minutes au reflux, on refroidit et on acidifie avec de l'acide chlorhydrique concentré jusqu'a pH nettement acide (1 à 2). On extrait avec 400 ml de chloroforme, on sèche la phase organique sur de sulfate de sodium anhydre et on évapore à sec sous pression réduite. On dissout le résidu huileux, constitué par l'acide [1-(2-oxo-3-tétrahydrothiénylcarbamoyl)éthylthio]acétique dans 100 ml d'acétone et on ajoute à la solution acétonique la quantité calculée de carbonate potassique dissous dans une très petite quantité d'eau. On filtre le précipité ainsi obtenu et on le cristallise dans 500 ml d'éthanol. On obtient ainsi 105 g de [1-(2-oxo-3-tétrahydrothiénylcarbamoyl)éthylthio]acétate de potassium; F. 202 à 204°C.

En remplaçant le carbonate de potassium par une quantité équivalente de carbonate de sodium, on obtient le sel de sodium correspondant.

Exemple 3

A une solution de 4 g d'hydroxyde de sodium dans 20 ml d'eau, on ajoute, sous atmosphère d'azote, 12 g de thioglycolate d'éthyle et, ensuite, 25,2 g de 3-(2-bromoprionamido)-2-oxo-tétrahydrothiophène. On chauffe 5 minutes au reflux, puis on refroidit, on extrait deux fois avec 20,1 de chloroforme, on sèche la phase organique sur de sulfate de sodium anhydre et on évapore à sec le solvant sous pression réduite. Par cristallisation de résidu dans éther diéthylique/éthanol, on obtient 24 g de [1-(2-oxo-3-tétrahydrothiénylcarbomoyl)éthylthio]acétate d'éthyle; F. 80 à 85°C.

De la même façon, à partir du 3-bromoacétamido-2-oxo-tétrahydrothiophéne, on obtient le [(2-oxo-3-tétrahydrothiénylcarbamoyl)-méthylthio]acétate d'éthyle.

Exemple 4

A une solution de 9,8 g de thioglycolate d'éthyle et de 16,9 g de 3-(3-chloropropiomamido)-2-oxo-tétrahydrothiophène dans 200 ml de diméthylsulfoxyde, on ajoute 5,4 g d'hydroxyde de potassium à 85% dissous dans une quantité d'eau minimale. Après 30 minutes à la température ambiante, on ajoute 500 ml d'eau et on extrait avec 200 ml de chloroforme. On sèche la phase organique sur du sulfate de sodium anhydre et on évapore le solvant sous pression réduite. Par cristallisation du résidu dans de l'isopropanol, on obtient 14 g de [2-(2-oxo-3-tétrahydrothiénylcarbamoyl)éthylthio]acétate d'éthyle; F. 59 à 60°C.

De la même façon, à partir de 5,34 g de 3-(2-bromobutyramido)-2-oxo-tétrahydrothiophène et 2,4 g de thioglycolate d'éthyle, on obtient, après cristallisation dans de l'éther diéthylique, 4,7 g de [1-(2-oxo-3-tétrahydrothiénylcarbamoyl)propylthio)acétate d'éthyle; F. 45 à 47°C.

Exemple 5

On ajoute lentement une solution de 22 g de triéthylamine dans 50 ml de toluène anhydre à une suspension, refroidie à —10°C, d'acide [(2-oxo-3-tétrahydrothiénylcarbamoyl)-méthylthio]acétique et 22 g de chlorocarbonate d'éthyle dans 500 ml de toluéne anhydre. On ajoute ensuite une solution de 19 g de phénol dans 200 ml de toluène anhydre, à la même température, puis on abandonne le mélange réactionnel 30 minutes à la température ambiante, on le filtre et on l'extrait d'abord avec une solution saturée de bicarbonate de sodium et ensuite avec de l'eau. On séche la phase organique sur du sulfate de sodium anhydre et on évapore le solvant sous pression réduite. Par cristallisation du résidu dans de l'éthanol, on obtient 51 g de [(2-oxo-3-tétrahydrothiénylcarbamoyl)-méthylthio]acétate de phényle; F. 99 à 101°C.

De la même façon, en faisant réagir 50 g d'acide [(2-oxo-3-tétrahydrothiénylcarbamoyl)-méthylthio]acétique, 22 g de triéthylamine et 22 g de chlorocarbonate d'éthyle, dans 500 ml de toluène anhydre, avec, respectivement 25 g de gaîacol et 30,8 g d'alcool p-nitrobenzylique, on obtient, respectivement, 52,5 g de [(2-oxo-3-tétrahydrothiénylcarbamoyl)méthylthio]acétate de 2-méthoxyphényle qui, après cristallisation dans l'éthanol, fond à 105—106°C et 57 g de [(2-oxo-3-tétrahydrothiénylcarbamoyl)méthylthio]acétate de p-nitrobenzyle qui, après cristallisation dans l'éthanol, fond à 99—100°C.

Exemple 6

On prépare un sirop ayant la composition suivante:

| | |
|---|---|
| PV 144 sel de sodium | 2 g |
| Saccharose | 70 g |

| | |
|---|---|
| p-hydroxybenzoate de méthyle | 0,15 g |
| Aromatisant | 1,25 g |
| Eau (q.s.p.) | 100 ml |

Exemple 7

On prépare un sirop ayant la composition suivante:

| | |
|---|---|
| PV 144 sel de sodium | 5 g |
| Saccharose | 40 g |
| p-hydroxybenzoate de méthyle | 0,15 g |
| Aromatisant | 2 g |
| Eau (q.s.p.) | 100 ml |

Exemple 8

On prépare des comprimés contenant 300 mg de PV 144 de la façon suivante: 60 kg de PV 144, 12 kg de lactose et 8 g d'amidon de maîs sont granulés avec 4 kg de polyvinylpyrrolidone (poids moléculaire environ 25.000) dans 6 litres d'eau et on les fait passer à travers un tamis de 1,25 mm de diamètre de mailles.

Après séchage, on ajoute 10 kg de carboxyméthylcellulose, 4 kg de talc et 2 kg de stéarate de magnésium. Sur une pastilleuse à tambour, on comprime le granulat en comprimés de 11 mm de diamètre et pesant 500 mg. De façon analogue, on prépare des comprimés contenant 150 et 500 mg de PV 144.

Exemple 9

10 000 gélules contenant 50 mg de substance active sont préparées à partir des constituants suivants: 500 g de PV 144, 495 g de cellulose microcristalline, 5 g de gel de silice amorphe sont bien mélangés et on les introduite dans des gélules de gélatine dure de dimension 4.

De la même façon, on prépare des gélules contenant 100 et 200 mg de PV 144.

**Revendications pour les Etats contractants: BE CH DE FR GB IT LI LU NL SE**

1. Acides [(2-oxo-3-tétrahydrothiénylcarbamoyl)-alkylthio]-acétiques, leurs sels et leurs esters de formule:

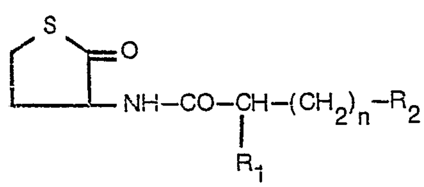

I

dans laquelle n représente 0 ou 1 et un des substituants $R_1$ et $R_2$ représente un atome d'hydrogène ou un groupe méthyle, et l'autre représente un groupe $-S-CH_2-COOR_3$, où $R_3$ est un atome d'hydrogène ou d'un métal alcalin, ou bien un groupe $R_3'$, $R_3'$ étant un alkyl comportant 1 à 3 atomes de carbone ou un groupe:

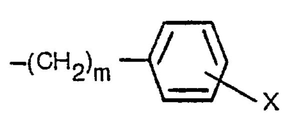

où m représente 0 ou 1 et X représente un atome d'hydrogène ou un groupe alkyle comportant 1 à 3 atomes de carbone, alcoxy comportant 1 à 3 atomes de carbone ou nitro.

2. Acide [(2-oxo-3-tétrahydrothiénylcarbamol)-méthylthio]-acétique de formule:

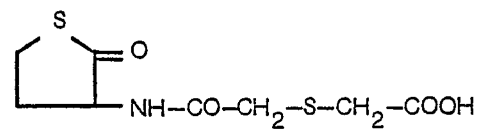

ainsi que ses sels de sodium et de potassium.

3. [(2-Oxo-3-tétrahydrothiénylcarbamoyl)-méthylthio]-acétate d'éthyle.

4. [(2-Oxo-3-tétrahydrothiénylcarbamoyl)-méthylthio]-acétate de phényle.

5. [(2-Oxo-3-tétrahydrothiénylcarbamoyl)-méthylthio]-acétate de 2-méthoxyphényle.

6. Procédé pour la préparation d'acides [(2-oxo-3-tétrahydrothiénylcarbamoyl)-alkylthio]-acétiques, leurs sels et leurs esters de formule:

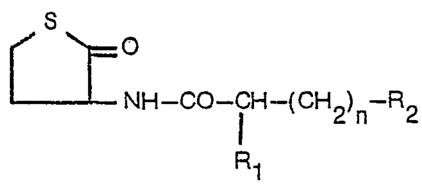

dans laquelle n représente 0 ou 1 et un des substituants $R_1$ et $R_2$ représente un atome d'hydrogène ou un groupe méthyle et l'autre représente un groupe $—S—CH_2—COOR_3$, où $R_3$ est un atome d'hydrogène ou d'un métal alcalin ou bien un groupe:

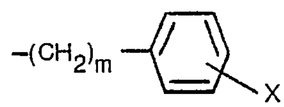

où m représente 0 ou 1 et X représente un atome d'hydrogène ou un groupe alkyle comportant 1 à 3 atomes de carbone, alcoxy comportant 1 à 3 atomes de carbone, ou nitro, caractérisé en ce que l'on fait réagir un 3-haloacylamino-2-oxo-tétrahydrothiophène de formule:

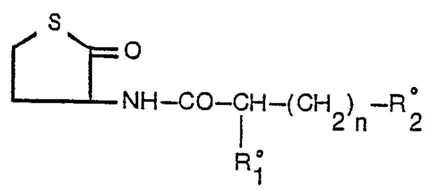

dans laquelle un des substituants $R_1^{\circ}$ et $R_2^{\circ}$ représente un atome d'hydrogène ou un groupe méthyle et l'autre représente un atome de chlore ou de brome, avec un acide thioglycolique de formule:

$$HS—CH_2—COOR_3^{\circ}$$

dans laquelle $R_3^{\circ}$ a la signification donnée ci-dessus pour $R_3$, mais est autre qu'un métal alcalin, en présence d'un accepteur de protons à une température de 15 à 120°C et, lorsque $R_3^{\circ}$ est de l'hydrogène, on transforme éventuellement le produit ainsi obtenu dans ses sels alcalins.

7. Procédé selon la revendication 6, caractérisé en ce que, comme produit de départ, on utilise l'acide thioglycolique libre.

8. Procédé selon les revendications 6 et 7, caractérisé en ce que le produit final acide libre est estérifié avec un composé de formule $R_3'$—OH, dans laquelle $R_3'$ est tel que défini dans la revendication 1.

9. Procédé selon les revendications 6 à 8, caractérisé en ce qu'on fait réagir un dérivé fonctionnel du produit final acide libre avec le composé de formule $R_3'$—OH, où $R_3'$ est tel que défini dans la revendication 1, à une température de −20 à +40°C, en présence d'un accepteur de protons dans un solvant organique inerte.

10. Procédé selon les revendications 6 à 9, caractérisé en ce qu'on fait réagir le produit final acide libre avec du chlorcarbonate d'éthyle et avec le composé de formule $R_3'$—OH, où $R_3'$ est tel que défini dans la revendication 1, en présence d'une base organique, à une température de −15 à −10°C.

11. Procédé selon la revendication 10, caractérisé en ce que, comme base organique, on utilise la triéthylamine.

12. Composition pharmaceutique renfermant, en tant qu'ingrédient actif, un composé selon la revendication 1 en mélange avec un excipient pharmaceutique.

13. Composition pharmaceutique selon la revendication 12, sous forme d'unité posologique, contenant de 1 à 1000 mg d'un composé selon la revendication 1 en mélange avec un excipient pharmaceutique.

14. Composition pharmaceutique selon la revendication 12, caractérisée en ce qu'elle contient, en tant qu'ingrédient actif, l'acide [(2-oxo-3-tétrahydrothiénylcarbamoyl)-méthylthio]-acétique ou son sel de sodium ou de potassium.

**Revendications pour l'Etat contractant: AT**

1. Procédé pour la préparation d'acides [(2-oxo-3-tétrahydrothiénylcarbamoyl)-alkylthio]-acétiques, leurs sels et leurs esters de formule:

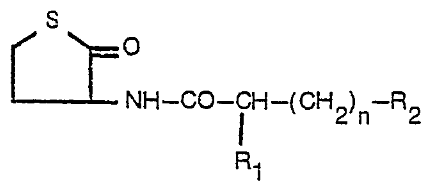

dans laquelle n représente 0 ou 1 et un des substituants $R_1$ et $R_2$ représente un atome d'hydrogène ou un groupe méthyle et l'autre représente un groupe $—S—CH_2—COOR_3$, où $R_3$ est un atome d'hydrogène ou d'un métal alcalin ou bien un groupe

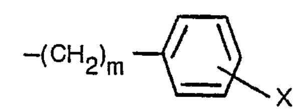

où m représente 0 ou 1 et X représente un atome d'hydrogène, ou un groupe alkyle comportant 1 à 3 atomes de carbone, alkoxy comportant 1 à 3 atomes de carbone, ou nitro, caractérisé en ce que l'on fait réagir un 3-halocylamino-2-oxo-tétrahydrothiophène de formule:

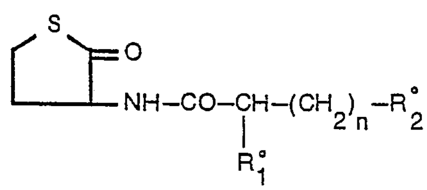

dans laquelle un des substituants $R_1^{\circ}$ et $R_2^{\circ}$ représente un atome d'hydrogène ou un groupe méthyle et l'autre représente un atome de chlore ou de brome, avec un acide thioglycolique de formule:

$$HS—CH_2—COOR_3^{\circ}$$

dans laquelle $R_3^{\circ}$ a la signification donnée ci-dessus pour $R_3$, mais est autre qu'un métal alcalin, en présence d'un accepteur de protons à une température de 15 à 120°C et, lorsque $R_3^{\circ}$ est de l'hydrogène, on transforme éventuellement le produit ainsi obtenu dans ses sels alcalins.

2. Procédé selon la revendication 1, caractérisé en ce que comme produit de départ on utilise l'acide thioglycolique libre.

3. Procédé selon l'une des revendications 1 et 2, caractérisé en ce que le produit final acide libre est estérifié avec un composé de formule $R_3—OH$, dans laquelle $R_3$ est tel que défini dans la revendication 1.

4. Procédé selon l'une des revendications 1 à 3, caractérisé en ce qu'on fait réagir un dérivé fonctionnel du produit final acide libre avec le composé de formule $R_3—OH$, où $R_3$ est tel que défini dans la revendication 1, à une température de −20 à +40°C en présence d'un accepteur de proton dans un solvant organique inerte.

5. Procédé selon l'une des revendications 1 à 4, caractérisé en ce qu'on fait réagir le produit final acide libre avec du chlorocarbonate d'ethyle et avec le composé de formule $R_3—OH$, où $R_3$ est tel que défini dans la revendication 1, en présence d'une base organique à une température de −15 à −10°C.

6. Procédé selon la revendication 5, caractérisé en ce que comme base organique on utilise la triéthylamine.

7. Composition pharmaceutique renfermant, en tant qu'ingrédient actif, un composé selon la revendication 1 en mélange avec un excipient pharmaceutique.

**Patentansprüche für die Vertragsstaaten: BE CH DE FR GB IT LI LU NL SE**

1. [(2-Oxo-3-tetrahydrothienylcarbamoyl)-alkylthio]-essigsäuren, ihre Salze und ihre Ester, mit der Formel:

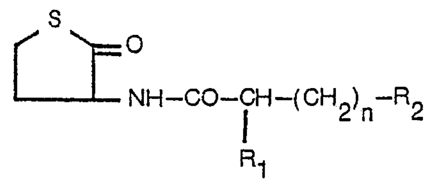

worin n für 0 oder 1 steht und einer der Substituenten $R_1$ und $R_2$ ein Wasserstoffatom oder eine Methylgruppe und der andere eine —S—$CH_2$—$COOR_3$—Gruppe darstellt, worin $R_3$ ein Wasserstoffatom oder ein Alkalimetallatom ist oder aber eine $R_3'$-Gruppe darstellt, wobei $R_3'$ ein Alkyl mit 1 bis 3 Kohlenstoffatomen oder eine Gruppe

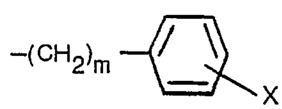

bedeutet, worin m für 0 oder 1 steht und X ein Wasserstoffatom oder ein Alkylgruppe mit 1 bis 3 Kohlenstoffatomen, eine Alkoxygruppe mit 1 bis 3 Kohlenstoffatomen oder eine Nitrogruppe bedeutet.

2. [(2-Oxo-3-tetrahydrothienylcarbamoyl)-methylthio]-essigsäure der Formel:

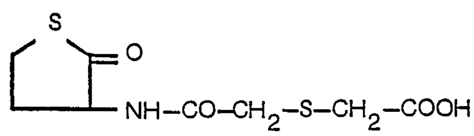

sowie ihre Natrium- und Kaliumsalze.

3. [(2-Oxo-3-tetrahydrothienylcarbamoyl)-methylthio]-äthylacetat.

4. [(2-Oxo-3-tetrahydrothienylcarbamoyl)-methylthio]-phenylacetat.

5. [(2-Oxo-3-tetrahydrothienylcarbamoyl)-methylthio]-2-methoxyphenylacetat.

6. Verfahren zur Herstellung von [(2-Oxo-3-tetrahydrothienylcarbamoyl)-alkylthio]-essigsäuren, ihren Salzen und ihren Estern, mit der Formel:

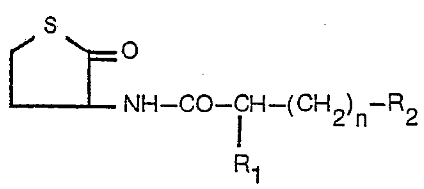

worin n für 0 oder 1 steht und einer der Substituenten $R_1$ und $R_2$ ein Wasserstoffatom oder eine Methylgruppe und der andere eine —S—$CH_2$—$COOR_3$—Gruppe darstellt, worin $R_3$ eine Wasserstoffatom oder ein Alkalimetallatom ist oder aber eine Gruppe

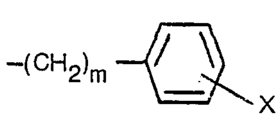

darstellt, worin m für 0 oder 1 steht und X ein Wasserstoffatom oder eine Alkylgruppe mit 1 bis 3 Kohlenstoffatomen, eine Alkoxygruppe mit 1 bis 3 Kohlenstoffatomen oder eine Nitrogruppe bedeutet, dadurch gekennzeichnet, daß ein 3-Haloacylamino-2-oxo-tetrahydrothiophen der Formel:

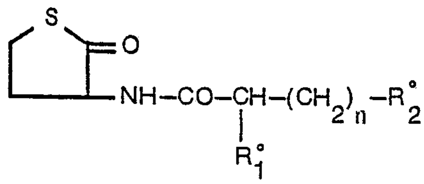

worin einer der Substituenten $R^\circ_1$ und $R^\circ_2$ ein Wasserstoffatom oder eine Methylgruppe darstellt und der andere ein Chlor- oder Bromatom bedeutet, mit einer Thioglykolsäure der Formel:

$$HS—CH_2COOR^\circ_3$$

worin $R^\circ_3$ die oben für $R_3$ angegebene Bedeutung hat, aber kein Alkalimetall ist, in Gegenwart eines Protonenakzeptors bei einer Temperatur von 15 bis 120°C zur Reaktion gebracht wird und für den Fall, daß $R^\circ_3$ Wasserstoff ist, das so erhaltene Produkt gegebenenfalls in seine Alkalisalze übergeführt wird.

7. Verfahren nach Anspruch 6, dadurch gekennzeichnet, daß als Ausgangsprodukt freie Thioglykolsäure verwendet wird.

8. Verfahren nach den Ansprüchen 6 und 7, dadurch gekennzeichnet, daß das freie Säure-Endprodukt mit einer Verbindung der Formel $R'_3$—OH, worin $R'_3$ die im Anspruch 1 angegebene Bedeutung hat, verestert wird.

9. Verfahren nach den Ansprüchen 6 bis 8, dadurch gekennzeichnet, daß ein funktionelles Derivat des freien Säure-Endprodukts mit der Verbindung der Formel $R'_3$—OH, worin $R'_3$ die im Anspruch 1 angegebene Bedeutung hat, bei einer Temperatur von —20 bis +40°C in Gegenwart eines Protonenakzeptors in einem inerten organischen Lösungsmittel zur Reaktion gebracht wird.

10. Verfahren nach den Ansprüchen 6 bis 9, dadurch gekennzeichnet, daß das freie Säure-Endprodukt mit Äthylchlorcarbonat und mit der Verbindung der Formel $R'_3$—OH, worin $R'_3$ die im Anspruch 1 angegebene Bedeutung hat, in Gegenwart einer organischen Base bei einer Temperatur von —15 bis —10°C zur Reaktion gebracht wird.

11. Verfahren nach Anspruch 10, dadurch gekennzeichnet, daß als organische Base Triäthylamin verwendet wird.

12. Pharmazeutische Zusammensetzung, die als aktives Ingredienz eine Verbindung nach Anspruch 1 in Mischung mit einem pharmazeutischen Inhaltsstoff enthält.

13. Pharmazeutische Zusammensetzung nach Anspruch 12 in Dosiseinheitsform, enthaltend 1 bis 1000 mg einer Verbindung nach Anspruch 1 in Mischung mit einem pharmazeutischen Inhaltsstoff.

14. Pharmazeutische Zusammensetzung nach Anspruch 12, dadurch gekennzeichnet, daß sie als aktives Ingredienz [(2-Oxo-3-tetrahydrothienylcarbamoyl)-methylthio]-essigsäure oder ihr Natrium- oder Kaliumsalz enthält.

**Patentansprüche für den Vertragsstaat: AT**

1. Verfahren zur Herstellung von [(2-Oxo-3-tetrahydrothienylcarbamoyl)-alkylthio]-essigsäuren, ihren Salzen und ihren Estern, mit der Formel:

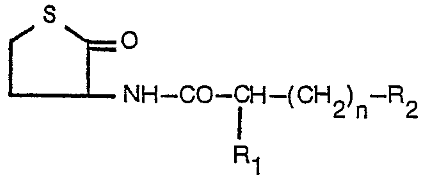

worin n für 0 oder 1 steht und einer der Substituenten $R_1$ und $R_2$ ein Wasserstoffatom oder eine Methylgruppe und der andere eine $—S—CH_2—COOR_3—$Gruppe darstellt, worin $R_3$ ein Wasserstoffatom oder ein Alkalimetallatom ist oder aber eine Gruppe

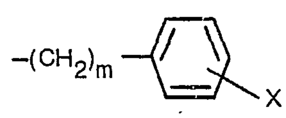

darstellt, worin m für 0 oder 1 steht und X ein Wasserstoffatom oder eine Alkylgruppe mit 1 bis 3 Kohlenstoffatomen, eine Alkoxygruppe mit 1 bis 3 Kohlenstoffatomen oder eine Nitrogruppe bedeutet, dadurch gekennzeichnet, daß ein 3-Haloacylamino-2-oxo-tetrahydrothiophen der Formel:

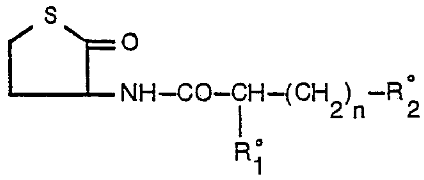

II

worin einer der Substituenten $R_1^{\circ}$ und $R_2^{\circ}$ ein Wasserstoffatom oder eine Methylgruppe darstellt und der andere ein Chlor- oder Bromatom bedeutet, mit einer Thioglykolsäure der Formel:

$$HS—CH_2—COOR_3^{\circ}$$

worin $R_3^{\circ}$ die oben für $R_3$ angegebene Bedeutung hat, aber kein Alkalimetall ist, in Gegenwart eines Protonenakzeptors bei einer Temperatur von 15 bis 120°C zur Reaktion gebracht wird und für den Fall, daß $R_3^{\circ}$ Wasserstoff ist, das so erhaltene Produkt gegebenenfalls in seine Alkalisalze übergeführt wird.

2. Verfahren nach Anspruch 1, dadurch gekennzeichnet, daß als Ausgangsprodukt freie Thioglykolsäure verwendet wird.

3. Verfahren nach einem der Ansprüche 1 und 2, dadurch gekennzeichnet, daß das freie Säure-Endprodukt mit einer Verbindung der Formel $R'_3$—OH, worin $R'_3$ die im Anspruch 1 angegebene Bedeutung hat, verestert wird.

4. Verfahren nach einem der Ansprüche 1 bis 3, dadurch gekennzeichnet, daß ein funktionelles Derivat des freien Säure-Endprodukts mit der Verbindung der Formel $R'_3$—OH, worin $R'_3$ die im Anspruch 1 angegebene Bedeutung hat, bei einer Temperatur von —20 bis +40°C in Gegenwart eines Protonenakzeptors in einem inerten organischen Lösungsmittel zur Reaktion gebracht wird.

5. Verfahren nach einem der Ansprüche 1 bis 4, dadurch gekennzeichnet, daß das freie Säure-Endprodukt mit Äthylchlorcarbonat und mit der Verbindung der Formel $R'_3$—OH, worin $R'_3$ die im Anspruch 1 angegebene Bedeutung hat, in Gegenwart einer organischen Base bei einer Temperatur von —15 bis —10°C zur Reaktion gebracht wird.

6. Verfahren nach Anspruch 5, dadurch gekennzeichnet, dass als organische Base Triäthylamin verwendet wird.

7. Pharmazeutische Zusammensetzung enthaltend, als aktives Ingredienz, eine Verbindung nach Anspruch 1 in Mischung mit einem pharmazeutischen Inhaltstoff.

**Claims for the Contracting States: BE CH DE FR GB IT LI LU NL SE**

1. [(2-oxo-3-tetrahydrothienylcarbamoyl)-alkylthio]-acetic acids, their salts and esters, of formula:

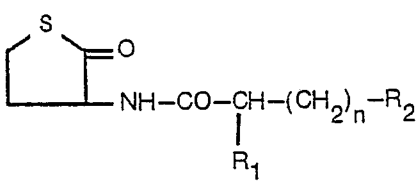

in which n represents 0 or 1 and one of the substituents $R_1$ and $R_2$ represents a hydrogen atom or a methyl group and the other represents a group $—S—CH_2—COOR_3$, where $R_3$ is a hydrogen atom or an alkali metal or a $R'_3$ group, $R'_3$ being an alkyl comprising between 1 to 3 carbon atoms or a group:

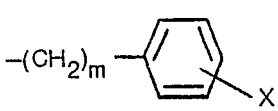

where m represents 0 or 1 and X represents a hydrogen atom or an alkyl group, comprising between 1 to 3 carbon atoms, alkoxy group comprising between 1 to 3 carbon atoms or a nitro group.

2. [(2-oxo-3-tetrahydrothienylcarbamoyl)-methylthio]-acetic acid of formula:

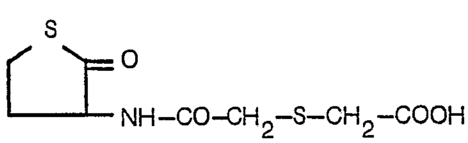

with their sodium and potassium salts.

3. [(2-Oxo-3-tetrahydrothienylcarbamoyl)-methylthio]-ethyl acetate.

4. [(2-Oxo-3-tetrahydrothienylcarbamoyl)-methylthio]-phenyl acetate.

5. [(2-Oxo-3-tetrahydrothienylcarbamoyl)-methylthio]-2-methoxyphenyl acetate.

6. Process for the preparation of [(2-oxo-3-tetrahydrothienylcarbamoyl)-alkylthio]-acetic acids, their salts and esters, of formula:

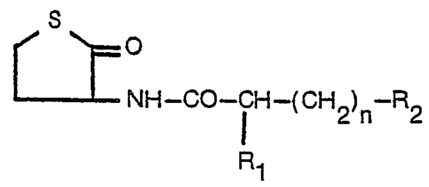

in which n represents 0 to 1 and one of the substituents $R_1$ and $R_2$ represents a hydrogen atom or a methyl group and the other represents a group —S—$CH_2$—$COOR_3$, where $R_3$ is a hydrogen atom or an alkali metal or a group

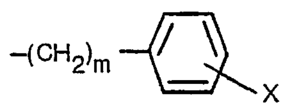

where m represents 0 to 1 and X represents a hydrogen atom or an alkyl group, comprising between 1 to 3 carbon atoms, alkoxy group comprising between 1 to 3 carbon atoms or a nitro group, characterized in that a 3-halocylamino-2-oxo-tetrahydrothiophene of formula

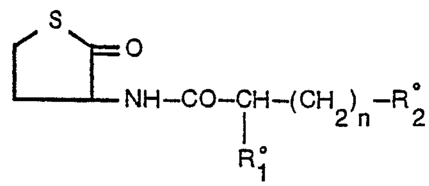

in which one of the substituents $R_1^\circ$ and $R_2^\circ$ represents a hydrogen atom or a methyl group and the other represents a chlorine or bromine atom, is reacted with a thioglycolic acid of formula:

$$HS—CH_2—COOR_3^\circ$$

in which $R_3^\circ$ has the meaning defined hereinabove for $R_3$, but is other than an alkali metal, in the presence of a proton acceptor at a temperature of 15 to 120°C, when $R_3^\circ$ is hydrogen and the product thus obtained is possibly converted into its alkaline salts.

7. The process according to claim 6, characterized in that the free thioglycolic acid is used as starting product.

8. The process according to claims 6 and 7 characterized in that the free acid final product is esterified with a compound of formula $R_3'$—OH in which $R_3'$ is as defined in claim 1.

9. The process according to claims 6 to 8, characterized in that a functional derivative of the free acid final product is reacted with the compound of formula $R_3'$—OH, where $R_3'$ is as defined in claim 1, at a temperature of −20 to +40°C in the presence of a proton acceptor in an inert organic solvent.

10. The process according to claims 6 to 9, characterized in that the free acid final product is reacted with ethyl chlorocarbonate and with the compound of formula $R_3'$—OH, where $R_3'$ is as defined in claim 1, in the presence of an organic base at a temperature of −15 to −10°C.

11. The process according to claim 10, characterized in that triethylamine is used as organic base.

12. Pharmaceutical composition containing, as active ingredient, a compound according to claim 1 mixed with a pharmaceutical excipient.

13. Pharmaceutical composition according to claim 12, in the form of a dose unit, containing from 1 to 1000 mg of a compound according to claim 1 mixed with a pharmaceutical excipient.

14. Pharmaceutical composition according to claim 12, characterized in that it contains, as active ingredient, [(2-oxo-3-tetrahydrothienylcarbamoyl)-methylthio]-acetic acid or its sodium or potassium salt.

**Claims for the Contracting State: AT**

1. Process for the preparation of [(2-oxo-3-tetrahydrothienylcarbamoyl)-alkylthio]-acetic acids, their salts and esters, of formula:

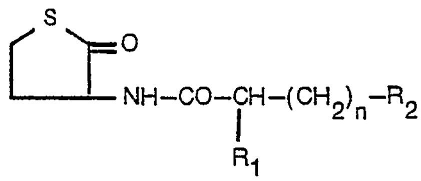

in which n represents 0 or 1 and one of the substituents $R_1$ and $R_2$ represents a hydrogen atom or a methyl group and the other represents a group $—S—CH_2—COOR_3$, where $R_3$ is a hydrogen atom or an alkali metal or a group

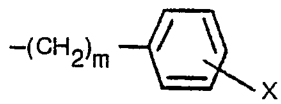

where m represents 0 or 1 and X represents a hydrogen atom or an alkyl group, comprising between 1 to 3 carbon atoms, alkoxy group comprising between 1 to 3 carbon atoms or a nitro group, characterized in that a 3-halocylamino-2-oxo-tetrahydrothiophene of formula

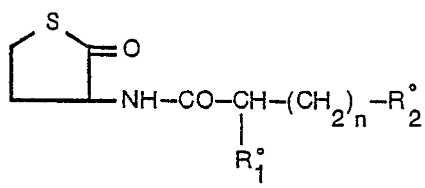

in which one of the substituents $R_1^\circ$ and $R_2^\circ$ represents a hydrogen atom or a methyl group and the other represents a chlorine or bromine atom, is reacted with a thioglycolic acid of formula:

$$HS—CH_2—COOR_3^\circ$$

in which $R_3^\circ$ has the meaning defined hereinabove for $R_3$, but is other than an alkali metal, in the presence of a proton acceptor at a temperature of 15 to 120°C, when $R_3^\circ$ is hydrogen and the product thus obtained is possibly converted into its alkaline salts.

2. The process according to claim 1, characterized in that the free thioglycolic acid is used as starting product.

3. The process according to claims 1 and 2, characterized in that the free acid final product is esterified with a compound of formula $R_3'—OH$ in which $R_3'$ is as defined in claim 1.

4. The process according to claims 1 to 3, characterized in that a functional derivative of the free acid final product is reacted with the compound of formula $R_3'—OH$, where $R_3'$ is as defined in claim 1, at a temperature of –20 to +40°C in the presence of a proton acceptor in an inert organic solvent.

5. The process according to claims 1 to 4, characterized in that the free acid final product is reacted with ethyl chlorocarbonate and with the compound of formula $R_3'—OH$, where $R_3'$ is as defined in claim 1, in the presence of an organic base at a temperature of –15 to –10°C.

6. The process according to claim 5, characterized in that triethylamine is used as organic base.

7. Pharmaceutical composition containing, as active ingredient, a compound according to claim 1 mixed with a pharmaceutical excipient.